# EUROPEAN PATENT APPLICATION

(11) **EP 1 374 688 A1**
(43) Date of publication of application: **02.01.2004**
(21) Application number: 02702758.0
(22) Date of filing: 06.03.2002
(51) Int. Cl.: A23B 7/00

(54) **ETHYLENE GAS ADSORBENT, METHOD OF CONTROLLING ETHYLENE GAS CONCENTRATION AND ETHYLENE GAS SENSOR**

(30) Priority: 08.03.2001 JP 2001064513; 08.03.2001 JP 2001064514; 08.03.2001 JP 2001064515
(71) Applicant: Keio University, Tokyo 108-8345 (JP)
(72) Inventor: SHIRATORI, Seimei, Yokohama-shi, Kanagawa 223-0061 (JP); HAYASHIBARA, Hitoshi, Ebina-shi, Kanagawa 243-0424 (JP)
(74) Representative: Wakerley, Helen Rachael
(86) International application number: PCT/JP2002/002070
(87) International publication number: WO 2002/071851

(57) **Abstract**

The present invention provides means which can be used for maintaining the freshness of or controlling maturation of plants or perishables such as fruits, vegetables, and flowers and which is safe to the human body. According to the present invention, amethod formaintaining the freshness of or conversely controlling the maturation of plants or perishables by controlling the concentration of ethylene gas by use of an ethylene gas adsorbent containing an alcohol extract solution of raw bamboo and a filter having held thereon the alcohol extract solution of raw bamboo . Also, according to the present invention, there is provided an ethylene gas sensor including a quartz crystal microbalance having coated thereon the alcohol extract solution of raw bamboo.

## Description

### TECHNICAL FIELD

The present invention relates to an ethylene gas adsorbent used for maintaining the freshness of plants and perishables such as fruits, vegetables, and flowers and to a wrapping material and a packaging material used for maintaining the freshness of plants and perishables such as fruits, vegetables, and flowers.

Also, the present invention relates to an ethylene gas concentration controlling method and apparatus used for maintaining the freshness of plants andperishables such as fruits , vegetables, and flowers or for controlling maturation thereof.

Further, the present invention relates to an ethylene gas sensor that detects ethylene in a gas and to a method and apparatus for measuring the concentration of ethylene gas in a gas.

### BACKGROUND ART

Fruits and vegetables such as apples and strawberries, or flowers such as carnation are usually packaged in a box in the producing district and transported therefrom. However, there has been a problem in that ethylene gas generated in the inside of, for example, fruits and released therefrom due to fermentation during transportation accelerates fermentation in other individuals or sites, so that the freshness of the transported fruits is decreased.

Accordingly, to transport fruits or other produce, in particular, to a distant location, a method must be adopted in which fruits or the like are harvested in an unripe state and delivered as they are to the hand of consumers while leaving the fruits to ripen during the transportation, so that it was difficult to ship the fruits in a completely ripened state and the delivered fruits in many cases tend to become poor in sugar degree or taste.

As means for solving this problem, a method in which potassium permanganate having a capability of decomposing ethylene gas is allowed to coexist during transportation or storage of fruits, vegetables, or the like to decompose and remove ethylene gas released from the fruits or vegetables has been proposed (JP 54-117060 A) and partly put to practical use. However, potassium permanganate has a problem of safety in that it is poisonous when it is swallowed or inhaled and it stimulates the mucous membrane or tissues strongly. Also, a method in which an adsorbent of the physical adsorption type, such as zeolite or activated carbon, is compounded in a wrapping material to thereby adsorb and remove ethylene gas has been proposed (JP 64-31838 A). However, the method exhibits no satisfactory effect. Further, a method in which vegetables are stored in a refrigerator in coexistence with a copper-containing antimicrobial agent has been also proposed (JP 11-211342 A). However, no satisfactory effect has been obtained in practical use. Furthermore, a method in which transportation is performed together with fruits having function of absorbing ethylene gas, such as banana and kiwi that are unripe, that is, in a green state, to prevent fermentation has been proposed (JP 11-32668 A). However, fermentation also proceeds in the fruits per se that absorb ethylene gas, so that prolonged effects cannot be expected. In addition, this method involves the risk of deteriorating the flavor of the fruit by the mixing of the scents due to mixing of different kind of fruits.

Further, JP 2000-210526 A discloses use of essential oils obtained by steam distillation of various kinds of plants as materials for removing hazardous gases and cites Japanese cypress and tea plant as examples of such plants. However, the essential oils obtained by steam distillation of these plants have had no fully satisfactory ability of removing ethylene gas.

Furthermore, as described above, it has been known that plants and perishables such as fruits, vegetables, and flowers generate ethylene gas as a result of fermentation and the released ethylene gas makes their fermentation proceed further. Accordingly, to control fermentation of fruits, vegetables, flowers and so forth andmaintain their freshness, it is important to detect ethylene gas in refrigerators and transportation chambers and measure its concentration.

Conventionally, gas sensors are directed to combustible gases, oxygen, humidity, and hazardous gases such as CO and have come to have a big market. Still now, new needs are created one after another and active researches are being carried out continuously. One recent big need is for detection of scent. Detection of scent finds specific applicationsin risk management, food management and the like and at the same time is a part of sensing of sensibilities, which is a new theme of information science.

A wide variety of scent components to be detected have been studied, examples of which include trimethylamine (TMA) and dimethylamine (DMA) that relate to the freshness of fish and shellfish; methylpyrazine, acetone and the like that are contained in consommé soup; ethyl acetate, acetoin and the like that relate to the freshness of beef; H₂S and NH₃ that are also hazardous gases; fruit oils, methylmercaptan, aliphatic amines and so forth. However, in actuality, substantially no study has been made for the detection of ethylene gas.

Conventional materials that have been known as materials for use in scent sensors include metal oxide semiconductors and organic materials. In sensors with metal oxide semiconductors, in which sintered compacts of oxide particles are mainly used, adsorption or reaction of scent molecules on or with the surface of the semiconductor particles changes the electrical resistance of the semiconductor, the amount of which change is taken out as a sensor signal. On the other hand, where organic materials are used, adsorption of the scent components on the organic materials changes the mass, absorbance or the like, the amount of which change is physically measured.

On the other hand, a method of using a quartz crystal microbalance as a sensor using an organic material (Quartz Crystal Microbalance method (QCM method)) has been known (G. Sauerbrey, "Verwendung von Schwingquartzen zur Wägung dünner Schichten und zur Mikrowägung", Zeitshrift für Physik, Vol. 155, pp.206-222, 1959). In this method, a quartz crystal microbalance is used as a high-sensitivity gas sensor element since the quartz crystal microbalance can detect a minute change in mass on an electrode of the quartz crystal microbalance due to, for example, gas adsorption thereon as a change in resonant frequency due to mass-loading effect. Further, the QCM method can easily realize sensors having various characteristics by varying the sensitive film to be coated on the electrode. As the film material, polymers and lipids are mainly used. In the case where scents are distinguished with the sensors, a technique of pattern recognition by multivariate analysis or through a neural network is used for the recognition of outputs from a plurality of sensors coated with different sensitive films.

As described above, although hazardous gas sensors or scent sensors have been conventionally developed, no simple and accurate ethylene gas sensor has been developed yet which is suitable for detecting and measuring concentration of ethylene gas in the gas in a refrigerator or transportation chamber in order to control fermentation of fruits, vegetables, and flowers therein to maintain their freshness.

### DISCLOSURE OF THE INVENTION

The present invention has been made to solve the above problems of the prior art and therefore an object of the present invention is to provide means that is safe to human body and used for maintaining the freshness of plants and perishables such as fruits, vegetables, and flowers for a long period of time. Further, another object of the present invention is to provide means for accelerating or conversely inhibiting maturation of plants and perishables such as fruits, vegetables, and flowers.

The inventors of the present invention have made extensive studies on the above-mentioned problems and as a result they have anticipated that use of ethylene monooxygenase that can biochemically metabolize and decompose ethylene gas in place of a physical adsorbent such as zeolite would solve the above-mentioned problems, thereby achieving the present invention. Then, the inventors of the present invention have focused attention, in particular, to an alcohol extract solution of raw bamboo as a substance containing ethylene monooxygenase.

That is, in the course of their studies on activated carbon (referred to as carbonized bamboo) obtained by roasting raw green bamboo (hereinafter simply referred to as raw bamboo) used for adsorption of ethylene gas, the inventors of the present invention have found that the raw bamboo itself adsorbs ethylene gas and conceived that use of ethylene monooxygenase, which is an ethylene oxidase contained in raw bamboo, and further an alcohol extract solution of raw bamboo would enable adsorption of ethylene gas more efficiently, thereby achieving the present invention.

In more detail, the present invention relates to an ethylene gas adsorbent characterized by containing ethylene monooxygenase.

It is preferable that the ethylene gas adsorbent of the present invention further contains a coenzyme (NADPH) in addition to ethylene monooxygenase.

The present invention further relates to an ethylene gas adsorbent using raw bamboo fiber or bamboo vinegar that contains ethylene monooxygenase, in particular an alcohol extract solution of raw bamboo. As bamboo vinegar, in particular an alcohol extract solution of raw bamboo is preferable.

Further, it is preferable that the ethylene gas adsorbent of the present invention is held on a substrate such as a transparent sheet-form material and that the ethylene gas adsorbent is held on the substrate through a gel forming substance having an amino group.

The present invention further relates to application of such an ethylene gas adsorbent to a wrapping material or a packaging material for wrapping or packaging plants and perishables such as fruits, vegetables, and flowers.

In the case where ethylene monooxygenase in the raw bamboo or the like of the present invention is used for the adsorption of ethylene gas, the present invention has an excellent advantage in that the ethylene monooxygenase causes no danger at all and is safe even if it contacts with foods and the like and introduced into the body of an individual or if it is eaten together with the foods since only nonhazardous plant-based materials are utilized.

Further, the present invention relates to a method for controlling the concentration of ethylene gas with a filter that ethylene monooxygenase is supported thereon. As the substance that contains ethylene monooxygenase, in particular an alcohol extract solution of raw bamboo is preferable.

It is preferred that said filter further contains NADPH as a coenzyme for ethylene monooxygenase.

Further, the present invention relates to a method for controlling a concentration of ethylene gas with a filter on which a raw bamboo fiber or bamboo vinegar solution. As the bamboo vinegar solution, in particular an alcohol extract solution of raw bamboo is preferable.

In order to control the concentration of ethylene gas, according to the present invention, the method for controlling the concentration of ethylene gas preferably includes measuring an ethylene gas concentration of an atmosphere by an ethylene gas concentration measuring apparatus; and decreasing the ethylene gas concentration by operating a fan to have ethylene gas adsorbed onto the filter when the ethylene gas concentration is equal to or above a predetermined value or introducing ethylene gas into the atmosphere when the ethylene gas concentration is equal to or below a predetermined value.

The present invention relates to a method for maintaining freshness or controlling maturation of perishables such as fruits, vegetables, and flowers , by using the above method for controlling ethylene gas concentration.

Further, the present invention relates to an apparatus for controlling ethylene gas concentration which includes the filter described above and a fan and also relates to an apparatus for maintaining freshness or controlling maturation of perishables such as fruits, vegetables, and flowers which is characterized by including an ethylene gas concentration measuring apparatus, the filter described above, and a fan.

Furthermore, the present invention relates to an ethylene gas sensor that detects ethylene gas in a gas as well as a method and apparatus for measuring the concentration of ethylene gas with said ethylene gas sensor.

That is, the present invention relates to an ethylene gas sensor characterized by including a quartz crystal microbalance having held thereon ethylene monooxygenase, in particular an alcohol extract solution of raw bamboo.

It is preferable that the ethylene gas sensor of the present invention further contains a coenzyme (NADPH).

The present invention also relates to an ethylene gas sensor including a quartz crystal microbalance having coated thereon bamboo vinegar solution containing ethylene monooxygenase.

Further, the present invention relates to an ethylene gas sensor, in which the quartz crystal microbalance is coated with the bamboo vinegar solution through a gel forming substance having an amino group.

The present invention also relates to a method and apparatus for measuring a concentration of ethylene gas in a gas by using the ethylene gas sensor described above.

### BRIEF DESCRIPTION OF THE DRAWINGS

Fig. 1 is a reference drawing for illustrating the rate of absorption of ethylene gas with respect to raw bamboo or carbonized bamboo.
Fig. 2 shows change over time of concentration of ethylene gas with respect to bamboo vinegar solution.
Fig. 3 is a schematic diagramof an ethylene gas concentration measuring apparatus of the present invention.
Fig. 4 shows adsorbability of ethylene gas by abamboo vinegar solution.
Fig. 5 shows adsorbability of ethylene gas with respect to an adsorbent holding a bamboo vinegar solution through a gel-forming substance.
Fig. 6 shows results of experiments of removing ethylene generated from an apple by a bamboo vinegar solution obtained by carbonization.
Fig. 7 shows results of experiments of removing ethylene generated from an apple by a bamboo vinegar solution obtained by extraction.
Fig. 8 shows a relationship between ethylene gas adsorbability of a bamboo vinegar solution obtained by extraction and a substrate.
Fig. 9 shows a change in concentration of ethylene gas in a sealed case in which an apple was placed according to Example 8.
Fig. 10 shows an ethylene gas concentration controlling apparatus of Example 10.
Fig. 11 shows a change in ethylene gas concentration in a storage chamber according to Example 10.
Fig. 12 shows the amount of ethylene gas adsorption with respect to the ethylene gas concentration, which is measured with the sensor of the present invention.
Fig. 13 shows comparison between the amount of ethylene gas adsorption and the amount of adsorption of other scent gas measured with the sensor of the present invention.

### BEST MODE FOR CARRYING OUT THE INVNETION

Hereinafter, the present invention will be described in detail.

The mechanism of ethylene metabolism in a plant tissue is described below. In "Mechanism of CH₂CH₂ metabolism in a plant tissue", [O] indicates ethylene oxidase (ethylene monooxygenase) As described below, there are two active sites for ethylene metabolism in a plant body. At an active site I, ethylene is oxidized and converted into ethylene oxide, which is further oxidized through ethylene glycol into carbon dioxide. At an active site II, after conversion into ethylene glycol, the ethylene glycol is combined with glucose to form ethylene glycol-glucose combination.

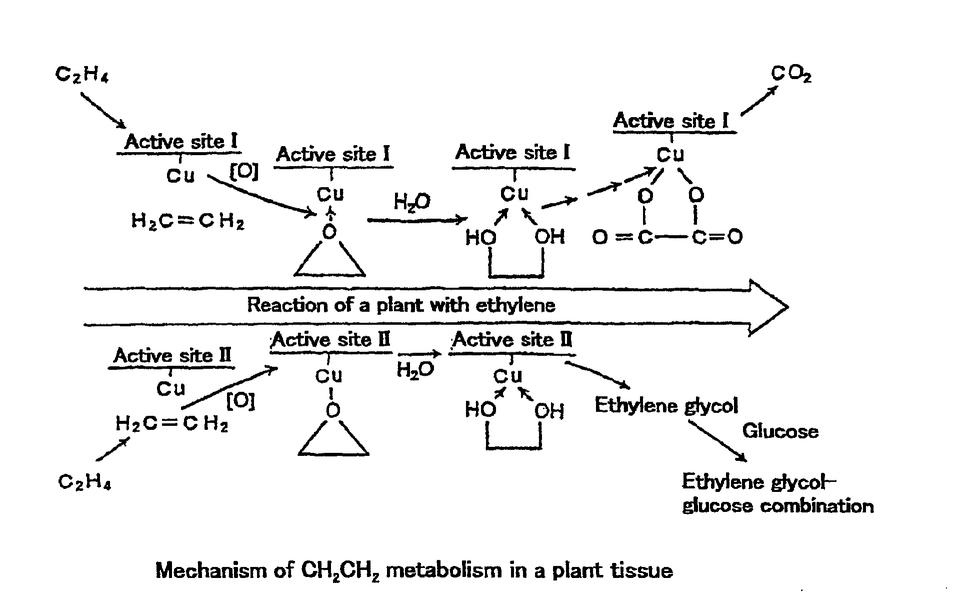

In the following description, the mechanism of ethylene metabolism is shown in more detail.

Ethylene is converted by an ethylene oxidase (ethylene monooxygenase) into ethylene oxide, which combines with a receptor to cause a biochemical reaction. On this occasion, NADPH is required as a coenzyme. The chemical reaction is carried out by utilizing energy released when the coenzyme is oxidized and decomposed. Further, ethylene oxide is converted into ethylene glycol at another site and metabolized into carbon dioxide. The carbon dioxide has an action of increasing ethylene monooxygenase activity. Glyoxylation of ethylene glycol is a route of detoxication of ethylene (cf., Keishi Shimokawa, "Ethylene", University of Tokyo Press (1988), p. 76 and 80).

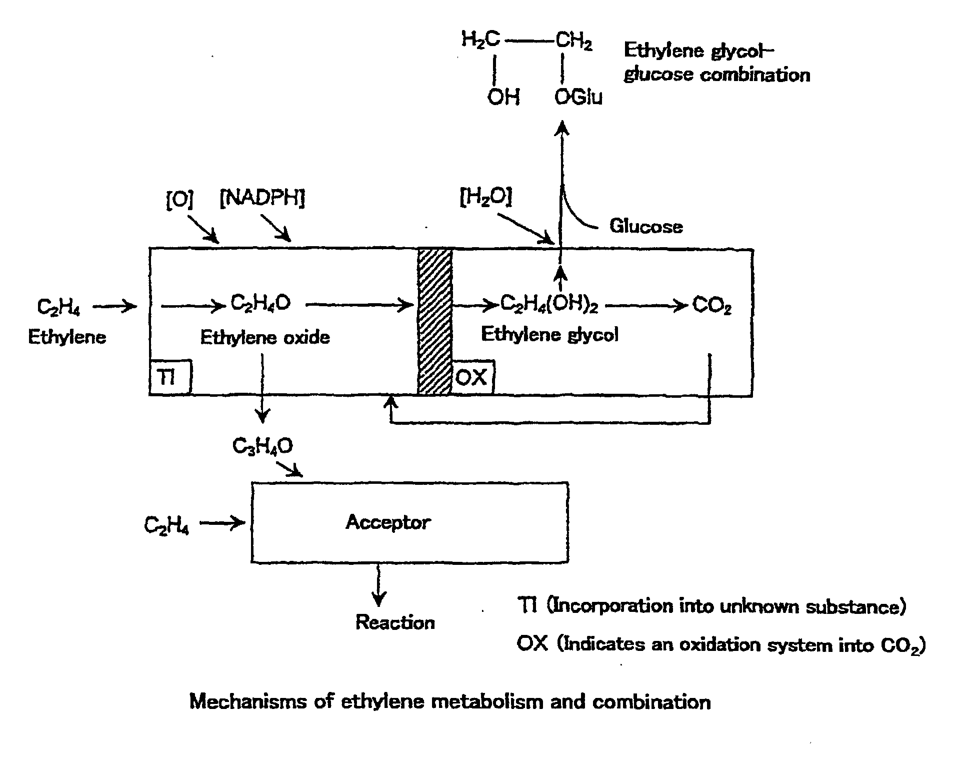

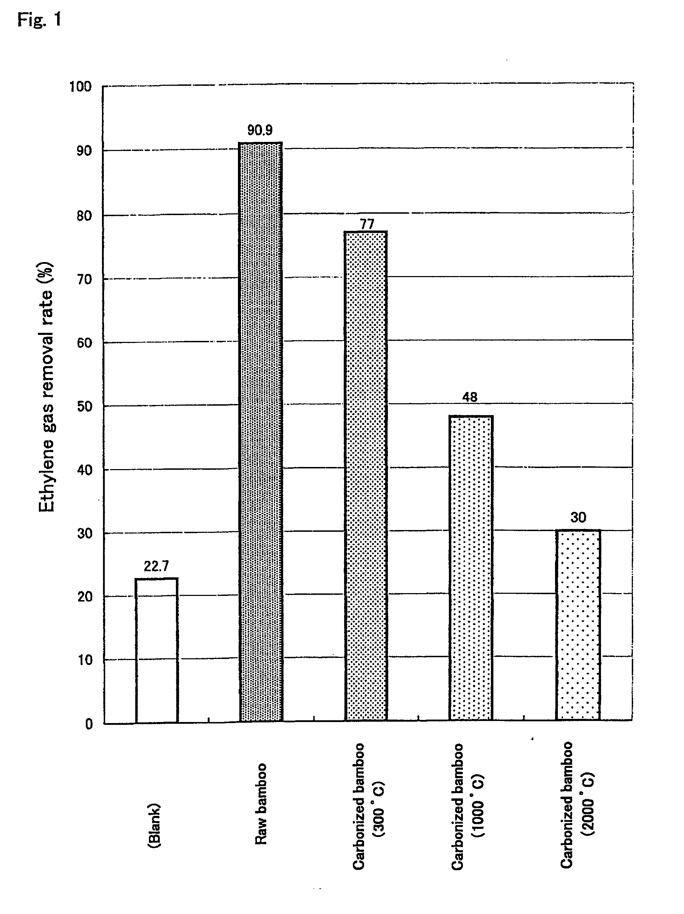

Initially, the inventors of the present invention have carried out tests of using raw bamboo cut laterally without any modification and activated carbon obtained by roasting said cut raw bamboo as ethylene gas adsorbents for maintaining the freshness of plants such as fruits , vegetables and flowers. For the purpose, 25 g each of raw bamboo and activated carbons (carbonized bamboo) obtained by roasting raw bamboo at 300°C, 1,000°C and 2,000°C, respectively, was charged in a sealed vessel filled with ethylene gas of a concentration of 50 ppm and air was circulated therein with a fan to carry out ethylene gas removal tests. Fig. 1 shows the results of the ethylene gas removal tests. From the results shown in Fig. 1, it has been recognized that raw bamboo removed ethylene gas best. This has led to anticipation that removal of ethylene gas may be related not to physical adsorption by activated carbon but to plant metabolism of bamboo.

However, because of poor usability provided by use of raw bamboo as it is for the adsorption of ethylene gas, studies have been made to see if there is a substance that is liquid allowing thin film formation and has an ethylene gas removing effect equivalent to that of raw bamboo. As a result, attention has been focused on bamboo vinegar obtained by dry distillation (carbonization) or extraction of raw bamboo.

In the present invention, bamboo vinegar is meant a liquid obtained from raw bamboo by dry distillation or extraction, more specifically, a liquid obtained by dry distillation of powdered raw bamboo at a temperature of 100 - 300°C with applying a pressure of 3 to 5 atm or a liquid obtained by extracting powdered raw bamboo with alcohol, water or an aqueous solution of an enzyme such as cellulase. In the present invention, it is most preferable that an alcohol extract solution obtained by extracting raw bamboo with an alcohol be used as a bamboo vinegar solution.

Next, the inventors of the present invention have tried to perform ethylene gas removal experiments with a bamboo vinegar solution obtained from raw bamboo. 100 ml of a bamboo vinegar solution obtained by dry distillation of raw bamboo was diluted with 300 ml of ultrapure water, and biodegradable resin foam particulates made of cellulose as a main ingredient were immersed therein for 24 hours and then spontaneously dried for 24 hours to fabricate a filter. As the biodegradable resin foam particulates were used particulates formed by pulverizing foam of a thumb size so as to have a large specific surface area. The fabricated filter was introduced in a sealed vessel filled with about 50 ppm of ethylene and air was circulated with a fan. After three hours, ethylene gas removal rate was measured. Table 1 shows the results obtained.

**<Table 1>**

| Ethylene gas removal rate | | |
|---|---|---|
| | Initial concentration | Concentration after three hours |
| Bamboo vinegar solution/foam | 55 ppm 55 ppm | 0.2 ppm 0.2 ppm |
| Foam only | 58 ppm | 21.0 ppm |

From the results, it can be seen that the foam immersed in the bamboo vinegar solution considerably increased the removal rate as compared with the foam alone.

As described above, in a plant body, ethylene is converted into ethylene oxide by an ethylene oxidase (ethylene monooxygenase) and further into ethylene glycol and finally metabolized into carbon dioxide. From this and the above-mentioned test results obtained by the inventors of the present invention that a radial section of raw bamboo or a bamboo vinegar solution exhibits the effect of adsorbing ethylene gas, the inventors of the present invention have presumed that catabolism (destructive metabolism) of ethylene with an enzyme contained in raw bamboo or a bamboo vinegar solution may be involved in the ability of removing ethylene gas by raw bamboo or a bamboo vinegar solution.

On the other hand, the inventors of the present invention have analyzed raw bamboo and a bamboo vinegar solution and as a result they have confirmed that the raw bamboo or the bamboo vinegar solution contained ethylene monooxygenase belonging to ethylene oxidase.

From the above-mentioned tests and analyses, the inventors of the present invention have obtained the knowledge that any material that contains ethylene monooxygenase, not limited to raw bamboo or a bamboo vinegar solution, is effective for adsorbing ethylene gas.

Further, based on the following experiments, the inventors of the present invention have found that among various kinds of plant extract solutions , an alcohol extract solution of raw bamboo is particularly suitable for the adsorption of ethylene gas. That is, the inventors of the present invention have carried out ethylene gas removal tests with alcohol solutions of Japanese cypress and of a tea plant and compared the results thereof with those of an alcohol solution of raw bamboo. Here, a liquid obtained by extracting 100 g of powder of each plant charged in a vessel with 70 cc of ethanol was used as an extract solution. As an adsorbent, used was one obtained by holding each extract solution on a substrate made of a biodegradable foam through chitosan that is a gel forming substance having an amino group. Table 2 shows the results of measurement of ethylene gas concentration after five days from introduction of each adsorbent in a sealed vessel filled with 100 ppm of ethylene gas.

**<Table 2>**

| Ethylene gas removing ability | | | |
|---|---|---|---|
| Adsorbent | Initial concentration (ppm) | After five days (ppm) | removal rate (%) |
| Bamboo extract solutions /chitosan /substrate | 100 | 40 to 50 | 50 to 60 |
| Japanese cypress /chitosan /substrate | 100 | 95 | 5 |
| Tea plant /chitosan /substrate | 100 | 100 | 0 |

From Table 2 , it can be seen that the alcohol extract solution of raw bamboo exhibits an ability of removing ethylene gas 10 times or more in comparison with those of the alcohol extract solutions of Japanese cypress or tea.

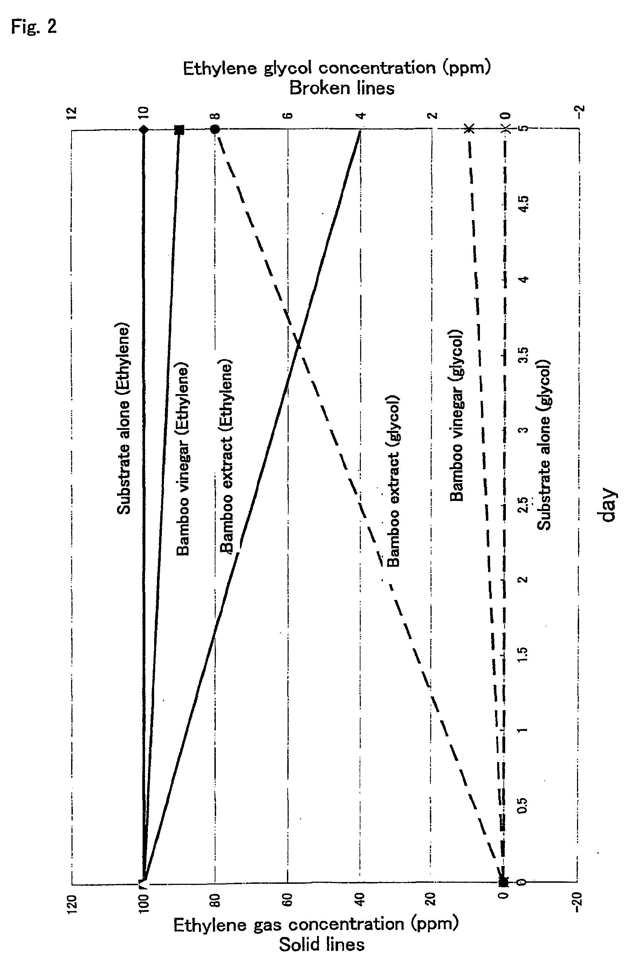

Next, the inventors of the present invention have compared the alcohol extract solution of raw bamboo with the dry distillate of raw bamboo with respect to their ability of ethylene gas adsorption. Fig. 2 shows the results. In Fig. 2, an alcohol extract solution of raw bamboo is represented as bamboo extract and a dry distillate of raw bamboo is represented as bamboo vinegar. In Fig. 2, the solid lines indicate abilities of ethylene gas removal, with the horizontal axis indicating number of days and the vertical axis indicating ethylene gas concentration. From the results, it can be seen that the ability of ethylene gas adsorption is more excellent for the alcohol extract of raw bamboo than for the dry distillate of raw bamboo. Note that in Fig. 2, the concentration of ethylene glycol released as a result of adsorption and oxidation of ethylene gas is also described (broken lines). Also, a released amount of ethylene glycol is more in the alcohol extract solution than in the dry distillate.

In the ethylene gas adsorbent of the present invention, raw bamboo, a bamboo vinegar solution or the like that contains ethylene monooxygenase is preferably held on a substrate. The substrate may be in any form, such as a film, sheet, granule, or mass. Examples of the material used for the substrate include polyethylene, polystyrene, wood pulp, cellulose, polyvinyl chloride, polyvinylidene chloride, cellophane, and cardboard box material. They may be made of foams.

In particular, when the ethylene gas adsorbent of the present invention is used as a wrapping material for plants such as fruits , vegetables and flowers, the substrate is preferably a transparent film such as a polyethylene sheet that can be seen through and more preferably a cellophane paper in order to arouse willingness to purchase.

When the ethylene gas adsorbent of the present invention is used as a packaging material for plants such as fruits, vegetables and flowers, wood pulp or natural cellulose sheets are preferable as substrates.

To hold the bamboo vinegar solution on a substrate, the bamboo vinegar solution as it is may be applied onto the substrate by immersion, casting or coating. However, holding the bamboo vinegar solution on a substrate through a gel forming substance having an amino group is preferred since the ethylene gas adsorption rate is increased.

It is presumed that holding a bamboo vinegar solution on a substrate through a gel forming substance having an amino group results in an increased ethylene gas adsorption rate because an amino group can react with a carboxyl group of the enzyme protein in the bamboo vinegar solution to immobilize the enzyme.

A preferable substance as the gel forming substance having an amino group includes polyallylamine, polyethyleneimine and chitosan . Chitosan has two amino groups in the molecule and in addition is harmless so that it is more preferable. When chitosan is used, it is preferable that a weak acid harmless to the human body, such as acetic acid or citric acid, be added to chitosan to form a transparent solution before it can be used.

The bamboo vinegar solution and the gel forming substance may be mixed with each other and held on a substrate or the gel forming substance may be held on a substrate and the bamboo vinegar solution may be laminated on the gel forming substance so as to be held thereon.

For example, the adsorbent of the present invention can be produced by using a solution prepared by mixing a bamboo vinegar solution with an aqueous solution of polyallylamine or an aqueous solution of chitosan in citric acid and coating or immersing a substrate such as a cellophane paper with or in the solution. Further, the adsorbent of the present invention can also be produced by coating a substrate with a solution of polyallylamine or chitosan in citric acid, drying the coating, further coating a bamboo vinegar solution on the coating, and drying the resulting coating.

Thus far, the ethylene gas adsorbent of the present invention has been explained as one used for holding the freshness of plants such as fruits, vegetables and flowers but the ethylene gas adsorbent of the present invention can be used as a filter for removing ethylene gas as well.

In the present invention, a method for controlling ethylene gas concentration includes decreasing the ethylene gas concentration by having ethylene adsorbed on a filter to remove ethylene from the atmosphere or conversely, increasing the ethylene gas concentration by having ethylene released from the filter. Ethylene can be eliminated from the atmosphere when it is desired to control thematuration of perishables such as fruits, vegetables and flowers to maintain the freshness thereof, and conversely ethylene can be desorbed from the filter so as to release to the atmosphere when it is desired to accelerate the maturation.

In the ethylene gas filter of the present invention, the raw bamboo fiber, bamboo vinegar solution, or the like that contains ethylene monooxygenase is held on a filter substrate. The filter substrate may be in any form selected from film, sheet, nonwoven fabric, woven fabric, granules, or mass and the material used for the substrate includes polyethylene, polystyrene, wood pulp, cellulose, polyvinyl chloride, polyvinylidene chloride, artificial fibers, animal fibers, plant fibers and glass fibers. These may be foams.

To hold raw bamboo fibers on a substrate, raw bamboo fibers are mixed into a filter substrate such as a foam, nonwoven fabric, or the like, or are adhered onto a surface of the filter substrate.

To hold a bamboo vinegar solution on the filter substrate, the bamboo vinegar solution as it is may be applied onto a substrate by immersion, casting or coating. However, holding it on a substrate through a gel forming substance having an amino group is preferred since the adsorption rate of ethylene gas is increased.

In the present invention, when a bamboo vinegar solution is used in an ethylene gas sensor, the bamboo vinegar solution is held on a quartz crystal microbalance. To hold the bamboo vinegar solution on a quartz crystal microbalance, the bamboo vinegar solution as it is may be cast or coated onto the quartz crystal microbalance but, as well as in the case where the bamboo vinegar solution is used in an absorbent or a filter, it is preferred that the bamboo vinegar solution be held on the substrate through a gel forming substance having an amino group since the ethylene gas adsorption rate is elevated.

The bamboo vinegar solution and the gel forming substance may be mixed with each other and held on a quartz crystal microbalance or the gel forming substance may be held on a quartz crystal microbalance and the bamboo vinegar solution may be laminated on the gel forming substance so as to be held thereon.

For example, the sensor of the present invention can be produced by using a solution prepared by mixing a bamboo vinegar solution with an aqueous solution of polyallylamine or an aqueous solution of chitosan in citric acid and coating or casting a quartz crystal microbalance therewith. Further, the sensor of the present invention can also be produced by coating a quartz crystal microbalance with a solution of polyallylamine or chitosan in citric acid, drying the coating, further coating a bamboo vinegar solution on the coating, and drying the resulting coating.

Hereinafter, examples of the present invention will be described. However, the present invention should not be considered to be limited thereto.

### <Example 1>

### (Production of a sensor)

A bamboo vinegar solution was obtained by charging powder of moso bamboo (Phyllostachys pubescens) in a basket and dry distilling it at 300°C and at 5 atm for 4 hours. 0.2 µl of the bamboo vinegar solutionwas cast on an electrode of a quartz crystal microbalance and spontaneously dried for 24 hours to obtain a sensor.

### (Ethylene gas adsorption test)

The sample thus obtained was inserted in a sealed vessel in which the ethylene gas concentration was kept at a constant level of 150, 300, or 800 ppm, and after 100 seconds the sample was taken out into the air. The measurement was repeated three times.

### (Method of testing adsorption amount)

The adsorption amount of ethylene gas was measured by use of quartz crystal microbalance method (QCM method). The QCM method utilizes the phenomenon that the eigenfrequency of a quartz crystal microbalance is changed due to a change in mass attached to the electrode. When a trace substance is attached onto the electrode of a quartz crystal microbalance by gas adsorption or the like, these minute mass change can be detected as a change in resonant frequency due to a mass loading effect.

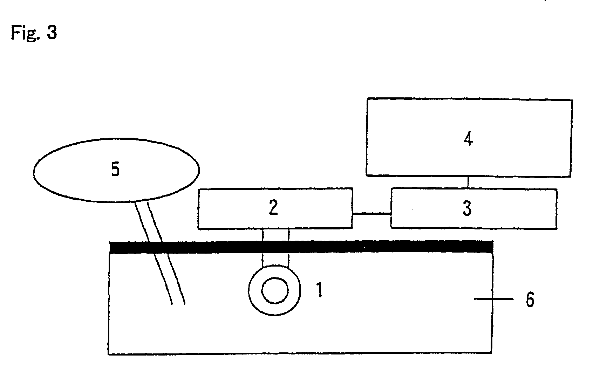

Fig. 3 shows a schematic diagram of an apparatus using the QCM method.

Reference numeral 1 designates a quartz crystal microbalance having cast thereon with ethylene monooxygenase, 2 designates an oscillation circuit, 3 designates a resonant frequency detector, and 4 designates a computer. A change in frequency appearing at 3 as a result of adsorption of ethylene gas supplied from an ethylene gas source 5 to a sealed vessel 6 onto the quartz crystal microbalance 1 is detected.

### (Results)

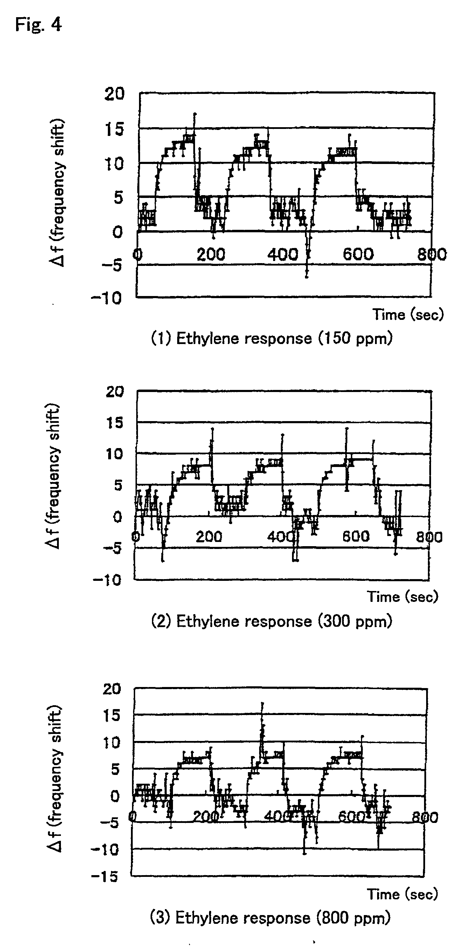

Fig. 4 shows the results. In Fig. 4, (1) shows the case in which the ethylene gas concentration was 150 ppm, (2) shows the case in which the ethylene gas concentration was 300 ppm, and (3) shows the case in which the ethylene gas concentration was 800 ppm. From those results, it can be seen that in any case in which the ethylene gas concentration was 150, 300 or 800 ppm, when the quartz crystal microbalance on which the bamboo vinegar solution was cast was inserted into the sealed vessel, adsorption of ethylene gas was started, while when the quartz crystal microbalance was exposed to the atmospheric air, the adsorbed ethylene was released. From this it was revealed that the quartz crystal microbalance having cast thereon with the bamboo vinegar solution could be used as an ethylene gas sensor. According to Figs. 4 (1) to (3) , the saturated adsorption amounts in respective cases are substantially equal and differences are minute.

### <Example 2>

In Example 2, ethylene gas adsorbent and a sensor comprising a composite film with a gel forming substance having an amino group are shown.

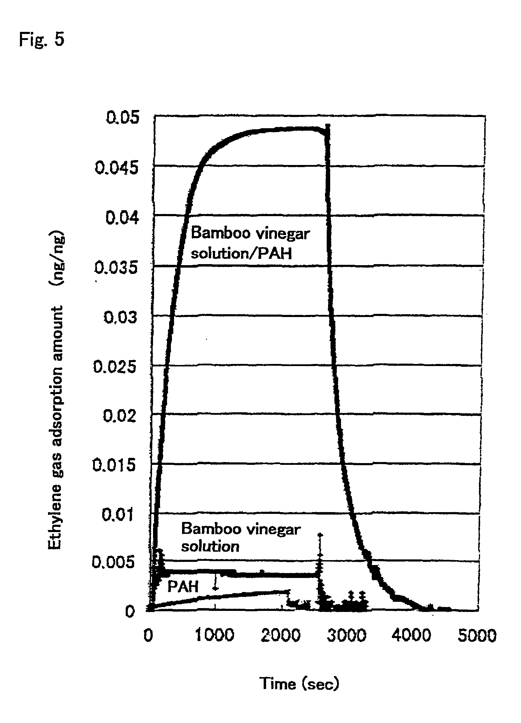

Before casting a bamboo vinegar solution on a quartz crystal microbalance, polyallylamine hydrochloride (PA) was used as a binder and the bamboo vinegar solution was cast thereon to prepare a composite film of PAH and the bamboo vinegar solution. First, the quartz crystal microbalance was subjected to hydrophilic treatment with KOH and 0.2 µl of a PAH solution was cast thereon and spontaneously dried for 24 hours to fabricate a thin film. Thereafter, 0.2 µl of the bamboo vinegar solution was cast and spontaneously dried for 24 hours to prepare a composite film. Quartz crystal microbalances with the prepared composite film, a single film of PAH or a single film of the bamboo vinegar solution , respectively was put into and out from a sealed vessel of which the ethylene concentration was 200 ppm and responses of adsorption and desorption were observed. Fig. 5 shows the results. In Fig. 5, the vertical axis indicates ethylene gas adsorption amount (ng/ng) per adsorbing film amount. From the results, it can be seen that a composite film with PAH increased the adsorbability as compared with the single film of the bamboo vinegar solution.

### <Example 3>

In Example 3, removal tests of ethylene generated from apple is shown.

Two acrylic resin cases of cubes of 200 mm in each side were provided and apples were placed therein. In one of them, 50 g of a biodegradable resin foam to which 30 cc of the bamboo vinegar solution obtained in Example 1 was attached and dried was arranged around the apples. On the other hand, in the other case, nothing was arranged therein. On the upper part of the cases were equipped fans respectively for circulating the air in these systems.

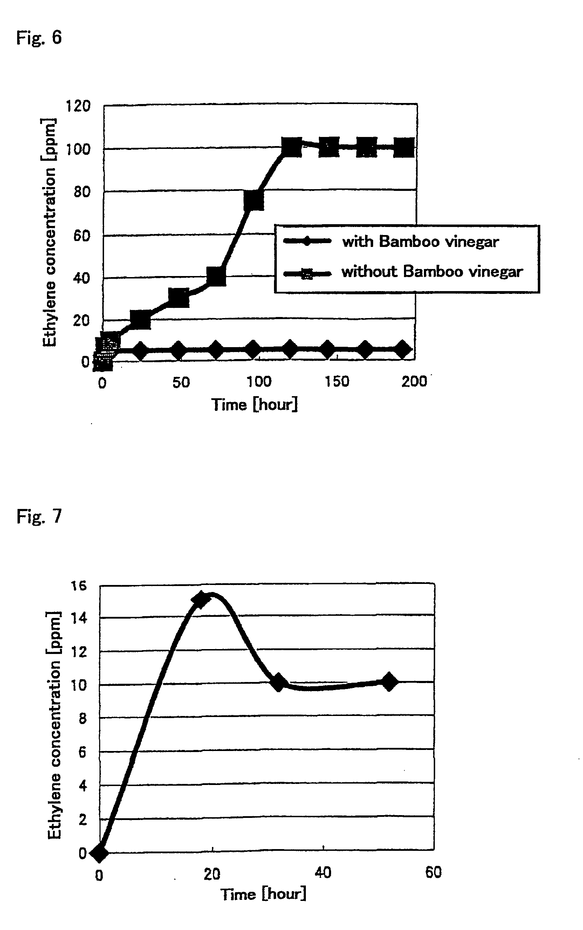

Fig. 6 shows the results of ethylene gas concentration measured after 192-hour from the start of the experiment by use of a gas detecting tube. The ethylene gas concentration in the case increased up to 100 ppm in the absence of the bamboo vinegar solution, due to generation of ethylene from the apples. On the other hand, in the presence of the bamboo vinegar solution, the ethylene gas concentration in the case was suppressed to 5 ppm. Thus, a difference in concentration was 20 times.

### <Example 4>

The experiment was carried out in the same manner as in Example 3 except that an extract solution obtained by extracting raw bamboo with an alcohol was used as a bamboo vinegar solution. As the bamboo vinegar solution, a liquid obtained by charging 100 g of powdered raw green moso bamboo in a vessel and extracting with 70 cc of ethanol was used.

Fig. 7 shows the results. In the case where the bamboo vinegar solution obtained from raw bamboo by extraction was used, the ethylene concentration in the case first increased up to 14 ppm but after about 30 hours, it was continually suppressed to about 10 ppm.

### <Example 5>

Ethylene gas adsorbability was examined by using an extract solution obtained by extracting raw bamboo with an alcohol as a bamboo vinegar solution and varying the substrate. Three kinds of substrate, i.e., a biodegradable foamed material, silica gel and activated carbon were used.

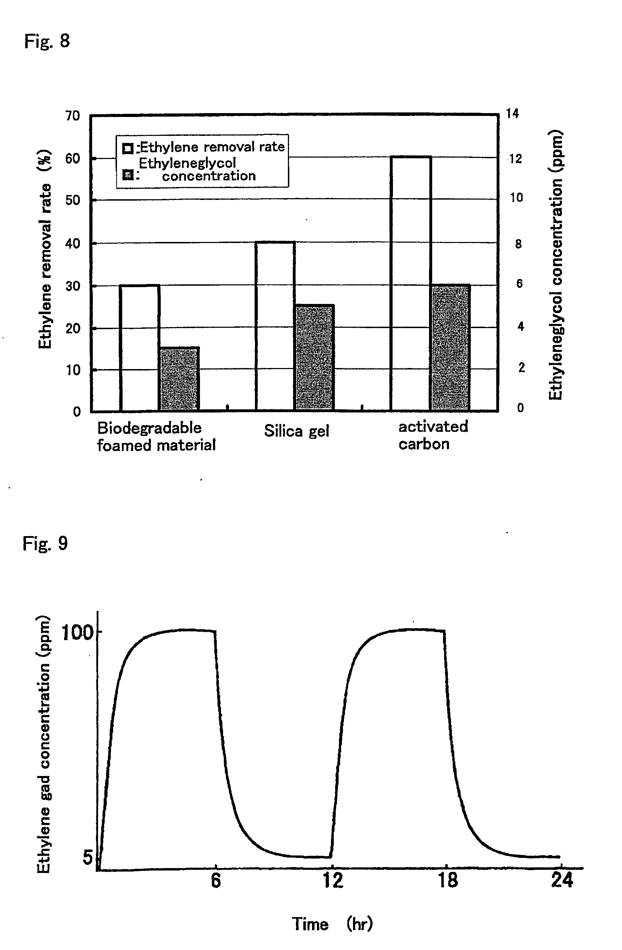

15 g of each substrate was immersed in 100 ml of an aqueous solution containing 1% chitosan for 10 minutes to treat the surface of the substrate. After annealing at 90°C for 2 hours, the substrate was immersed in 100 ml of the bamboo extract solution for 10 minutes and sufficiently dried spontaneously. 15 g of the adsorbent thus produced was charged in a 9-liter sealed vessel filled with 100 ppm of ethylene gas and ethylene gas was caused to be adsorbed while circulating the air by a fan. After 5 days, the removal rate of ethylene gas and the concentration of ethylene glycol produced were measured. Fig. 8 shows the results.

Since the specific surface area of the substrate increases in order of biodegradable foamedmaterial , silica gel and activated carbon, it can be seen from Fig. 8 that the removal rate of ethylene with the bamboo vinegar solution can be increased by increasing the specific surface are of the substrate.

### <Example 6>

An example in which the adsorbent of the present invention was used as a wrapping material of flowers is shown as follows.

As a wrapping material, cellophane paper coated with PAH to 2 g/m² and further coated with the bamboo vinegar solution obtained in Example 1 to 5 g/m² was used.

Six carnations in a bud stage were wrapped with the wrapping material and charged in a 30-liter acrylic resin case, which was then sealed and stored at 25°C for 3 days. During this period, the buds of carnation did not open at all and the size of the buds showed no change . However, when the carnations were taken out of the acrylic resin case, the buds were opened within 12 hours. This indicates that the wrapping material of the present invention can suppress the maturation of the flowers.

### <Example 7>

Shown is an example in which the adsorbent of the present invention was used as a packaging material.

The packaging material was obtained by coating a tray made of wood pulp and natural cellulose with a solution of chitosan in citric acid and further coating thereon with the bamboo vinegar solution (the same solution as in Example 1).

On this tray were mounted apples and these were placed in a sealed vessel and stored for 2 weeks while measuring the ethylene gas concentration in the vessel for every 24 hours.

The ethylene gas concentration in the vessel was suppressed to 5 ppm and the similar results as those in Fig. 6 were obtained.

### <Example 8>

Shown is an example in which the filter of the present invention was used for storage of perishables.

The filter of Example 1 was placed in an acrylic resin made sealed case of 200 mm in each side similar to that used in Example 1 and apples were arranged therein . The case was allowed to stand for 6 hours and the measured concentration of the generated ethylene gas was 100 ppm. The air in the sealed case was circulated by a fan through the filter, the ethylene gas concentration in the case was then decreased to 5 ppm. When the power of the fan was turned OFF and the case was allowed to stand for 6 hours again, the ethylene gas concentration in the sealed case increased to 100 ppm again. But when the fan was turned ON to circulate the air, the ethylene gas concentration was decreased to 5 ppm again. Fig. 9 shows a change of ethylene gas concentration in the sealed vessel.

In the case where the maturation of apples is intended to accelerate by use of the ethylene gas filter, the fan is kept OFF to increase the ethylene gas concentration, while in the case where the maturation of apples is intended to delay, the fan is turned ON to maintain the ethylene gas concentration at a low concentration.

### <Example 9>

Six roses in a bud stage were provided. Three of them were arranged in a usual room kept at 23°C. The remaining three were placed in a 1-m cubic vessel together with the filter holding the bamboo vinegar solution on a biodegradable resin foam. For roses kept in a usual room, two among three withered after 5 days, while they were still in the state of buds. On the other hand, for those kept in the vessel in which the bamboo vinegar solution was released, all the threewitheredafter theycompletelybloomed.

### <Example 10>

In Example 10, a system for controlling the ethylene gas concentration in the storage chamber of perishables by the feedback control type is shown.

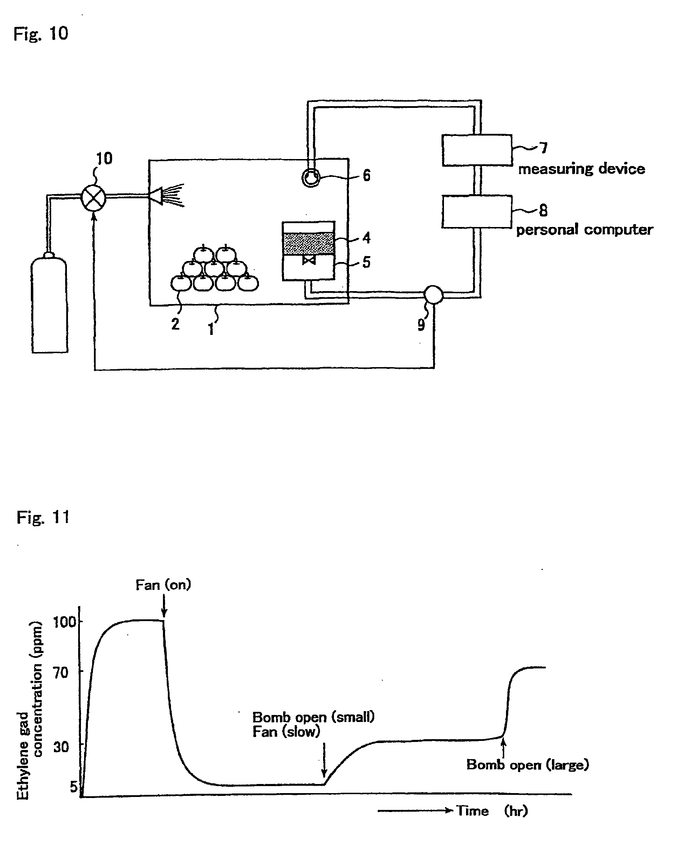

Fig. 10 shows a schematic diagram of the concentration control apparatus for a storage chamber. In Fig. 10, reference numeral 1 is a storage chamber for storing perishables such as fruits, 2 is perishables such as fruits to be stored, 3 is an ethylene gas bomb, 4 is an ethylene gas filter, 5 is a fan, 6 is an ethylene gas sensor, 7 is a measuring device, 8 is a personal computer, 9 is a feedback controller, and 10 is a solenoid valve.

Apples stored in the storage chamber 1 were kept in a low concentration ethylene gas atmosphere with turning the power of the fan 5 ON and having ethylene gas that was released from the apples adsorbed on the filter during the time when no order for purchase was received. When it was approached three days before the delivery date according to the purchase order received, the ethylene gas bomb was opened to introduce ethylene gas into the storage chamber so that the apples could be ripened with ease. During this time, the filter was rotated at a low speed so as to the ethylene gas concentration in the storage chamber became 30 ppm. Immediately before shipment, the ethylene gas concentration in the storage chamber was increased to 70 ppm and the apples were exposed to 70 ppm ethylene gas for 2 hours so as to they could be shipped after they were completely ripened.

Fig. 11 shows a change of ethylene gas concentration in the storage chamber by the ethylene gas concentration control system.

### <Example 11>

Next, application to a method and apparatus for measuring an ethylene gas concentration will be shown.

The sensor obtained in Example 2 with the composite film of PAH and the bamboo vinegar solution was placed in a sealed vessel filled with ethylene gas having a concentration of 8, 25, 30, 40, 200 or 600 ppm and its response was observed. The same ethylene gas measuring apparatus shown in Fig. 3 as in Example 1 was used. The ethylene gas concentration in the sealed vessel was measured by use of a commercially available detecting tube (manufactured by Gastec Corporation).

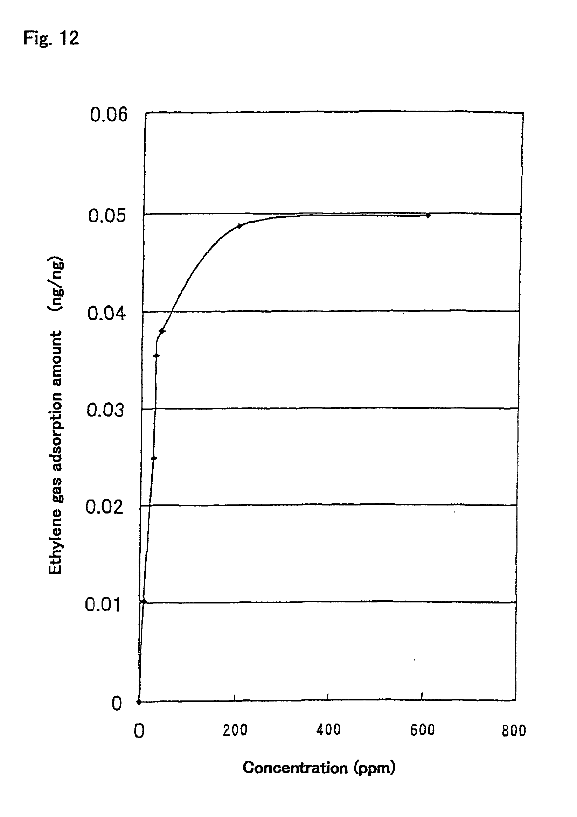

Fig. 12 shows the obtained results of adsorption isothermal curve. In Fig. 12, the horizontal axis indicates the ethylene gas concentration in the sealed vessel and the vertical axis indicates ethylene gas adsorption amount measuredby the QCMmethod. From Fig. 12 , it can be seen that the curve is a Langmuir adsorption isothermal. This has linearity at an ethylene gas concentration of 40 ppm or less and can be used as a simple ethylene gas concentration measuring apparatus having the same accuracy as that of the commercially available detecting tube. IF the ethylene gas concentration above 40 ppm, the curve is nonlinear but conversion using a calibration curve makes it possible to similarly use the apparatus in the measurement of concentrations .

### <Example 12>

Specificity of the ethylene gas sensor of the present invention to ethylene gas will be shown.

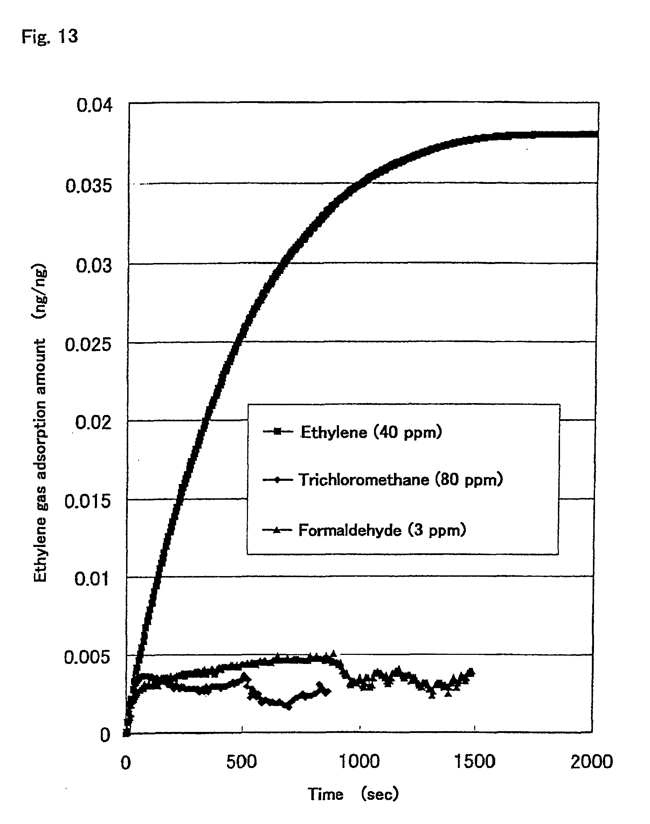

By using the QCM apparatus similar to that used in Example 1, 3 ppm of formaldehyde, 80 ppm of trichloromethane and 40 ppm of ethylene were filled in the sealed vessel. Fig. 13 shows the results. The horizontal axis indicates time and the vertical axis indicates adsorption amount of ethylene. From Fig. 13, it can be seen that the sensor of the present invention adsorbed ethylene gas but did not adsorb other compounds such as formaldehyde and trichloromethane. From the results, it can be seen that a sensor having adhered a bamboo vinegar solution in the form of a film onto a quartz crystal microbalance has a selectivity to ethylene gas.

### INDUSTRIAL APPLICABILITY

Ethylene gas adsorbent, wrapping material and packaging material containing ethylene monooxygenase, in particular an alcohol extract of raw bamboo can efficiently adsorb ethylene gas, so that they can suppress maturation by ethylene gas generated form plants such as fruits, vegetables and flowers.

Further, ethylene gas concentration can be safely controlled to the human body by use of a filter having supported thereon ethylene monooxygenase. Such a controlling method can be applied to maintaining freshness or controlling maturation of perishables such as fruits, vegetables and flowers in storage chamber or the like.

Furthermore, a quartz crystal microbalance having held thereon ethylene monooxygenase or a bamboo vinegar solution can selectively and efficiently adsorb ethylene gas so that it can be applied to a method and apparatus for measuring ethylene gas concentration.

## Claims

1. An ethylene gas adsorbent **characterized by** containing an alcohol extract solution of raw bamboo.

2. An ethylene gas adsorbent according to claim 1, wherein the ethylene gas adsorbent is held on a substrate.

3. An ethylene gas adsorbent according to claim 2, wherein the substrate is a transparent sheet-form material.

4. An ethylene gas adsorbent according to claim 2 or 3 , wherein the ethylene gas adsorbent is held on the substrate through a gel forming substance having an amino group.

5. A wrapping material or packaging material for plants, comprising the ethylene gas adsorbent according to any one of claims 2 - 4.

6. A method for controlling a concentration of ethylene gas with a filter having held thereon an alcohol extract solution of raw bamboo.

7. A method for controlling a concentration of ethylene gas, comprising measuring an ethylene gas concentration of an atmosphere by an ethylene gas concentration measuring apparatus; and decreasing the ethylene gas concentration by operating a fan to have ethylene gas adsorbed onto the filter according to claim 6 when the ethylene gas concentration is equal to or above a predetermined value or introducing ethylene gas into the atmosphere when the ethylene gas concentration is equal to or below a predetermined value.

8. A method for maintaining freshness or controlling maturation of perishables such as fruits, vegetables, and flowers, comprising using the method for controlling ethylene gas concentration according to claim 7.

9. An apparatus for controlling ethylene gas concentration, comprising the filter according to claim 6 and a fan.

10. An apparatus for maintaining freshness or controlling maturation of perishables such as fruits, vegetables, and flowers , comprising an ethylene gas concentration measuring apparatus, the filter according to claim 6, and a fan.

11. An ethylene gas sensor for sensing ethylene in a gas, **characterized by** comprising a quartz crystal microbalance having coated thereon an alcohol extract solution of raw bamboo.

12. An ethylene gas sensor according to claim 11, wherein the quartz crystal microbalance is coated with the alcohol extract solution of raw bamboo through a gel forming substance having an amino group.

13. A method for measuring a concentration of ethylene gas in a gas by use of the ethylene gas sensor according to claim 11 or 12.

14. A method for measuring a concentration of ethylene gas according to claim 13, wherein the concentration of ethylene gas is quantitatively determined by measuring a change in an eigenfrequency of the quartz crystal microbalance.

15. An apparatus for measuring the concentration of ethylene gas, **characterized by** including an ethylene gas sensor having an alcohol extract solution of raw bamboo coated on a quartz crystal microbalance; a device for measuring an eigenfrequency of the quartz crystal microbalance; a device for measuring a change in the eigenfrequency; and a device for converting a value of said change into an ethylene gas concentration.
